# EUROPÄISCHE PATENTANMELDUNG

(11) **EP 0 934 756 A2**
(43) Veröffentlichungstag der Anmeldung: **11.08.1999**
(21) Anmeldenummer: 99102063.7
(22) Anmeldetag: 28.01.1999
(51) Int. Cl.: A61M 27/00

(54) **Kapillar-Drainageschlauch-System**

(30) Priorität: 09.02.1998 DE 19805096
(71) Anmelder: PRIMED HALBERSTADT MEDIZINTECHNIK GMBH, 38820 Halberstadt (DE)
(72) Erfinder: Neubauer, Norbert, 38820 Halberstadt (DE)
(74) Vertreter: Pfeiffer, Rolf-Gerd, Dipl.-Phys.

(57) **Zusammenfassung**

Die Erfindung betrifft ein Kapillar-Drainageschlauch-System, welches für die Abführung von Körper- oder Wundsekreten Verwendung findet.
Die Aufgabe der Erfindung, ein Drainagesystem anzugeben, das die Nachteile des Standes der Technik vermeidet, insbesondere eine effektivere Sekretabführung ermöglicht, wird dadurch gelöst, daß in das distale Ende eines Schlauches (2) ein dieses Ende überragender Kapillarschlauch (1) eingeführt ist, wobei dem Kapillarschlauch (1) wenigstens auf seiner äußeren Oberfläche über den Umfang verteilt angeordnete, in Längserstreckungsrichtung zum Kapillarschlauch (1) verlaufende äußere Stege (111) gegeben sind, die voneinander derart beabstandet sind, daß sie in verlegten Zustand an ihrem äußeren Umfang vom Körpergewebe im wesentlichen umfaßt sind und somit ein Kapillarprofil (9) bilden.

## Beschreibung

Die Erfindung betrifft ein Kapillar-Drainageschlauch-System, welches für die Abführung von Körper- oder Wundsekreten Verwendung findet. Drainageschläuche werden seit Jahrzehnten für die Ableitung von Sekreten aus Operationswunden eingesetzt. Meistens handelt es sich dabei um flexible Schläuche aus Plastik oder Silicon, die steril an den zu versorgenden Ort im Körper des Patienten geführt sind.
Aus DE 88 13 995 U1 ist ein Drainageschlauch-System in Form eines dünnwandigen Flachprofils mit innen in Längsrichtung verlaufenden Rippen, an welches ein Schlauch mit Rundprofil ohne Innenrippen anvulkanisiert ist, bekannt.
In DE 33 25 920 wird ein Schlauch beschrieben, der mit vier außenseitigen rinnenartigen Vertiefungen versehen ist, wobei in den Vertiefungen Perforationslöcher eingebracht sind und der perforierte Teil mit einem enganliegenden, dünnwandigen und perforierten zweiten Schlauch überzogen ist, so daß das Wundsekret erst durch die Perforation des Überzugsschlauches und dann durch die Perforation der Vertiefungen des Grundschlauches fließt.
Darüber hinaus wird in DE 32 39 475 ein Schlauch angegeben, der mit einer Vielzahl von kleinen längsverlaufenden Rippen versehen ist, welche die Neigung des Schlauches zum Zusammenkleben während der Hitzesterilisation im Autoklaven herabsetzen und eine einfache Trennung eventuell zusammengeklebter Schlauchabschnitte nach dem Autoklavieren ermöglichen. Dieser Schlauch ist jedoch nicht als Drainageschlauch für den Wundsekretabfluß vorgesehen und geeignet.

Der Nachteil der beschriebenen Drainagesysteme besteht darin, daß alle Schläuche bzw. Schlauchteile, die in der Wunde liegen, perforiert oder geschlitzt sein müssen, so daß das Wundsekret erst einen Weg zum entsprechenden Perforationsloch oder Schlitz finden muß.

Der Erfindung liegt die Aufgabe zugrunde, ein Drainagesystem anzugeben, das die Nachteile des Standes der Technik vermeidet, eine effektive Sekretabführung gewährleistet und welches problemlos aus der Wunde derart entfernbar ist, daß eine zusätzliche Wundbehandlung unterbleiben kann.

Die Aufgabe wird durch die Merkmale des ersten Patentanspruchs gelöst. Vorteilhafte Ausgestaltungen sind von den nachgeordneten Ansprüchen erfaßt.

Die Erfindung soll nachstehend anhand schematischer Ausführungsbeispiele näher erläutert werden. Es zeigen:
- Fig. 1: eine erste Ausführungsmöglichkeit des erfindungsgemäßen Kapillar-Drainageschlauch-Systems,
- Fig. 2: einen Querschnitt des erfindungsgemäßen Kapillar-Drainageschlauch-System entlang einer Schnittebene A-A,
- Fig. 3: eine Befestigungsmöglichkeit des Kapillar-Drainageschlauch-System über einen an der Haut haftendem Sekretbeutel,
- Fig. 4: ein Kapillar-Drainageschlauch-System mit weiteren angeschlossenen Funktionseinheiten,
- Fig. 5: ein modifiziertes Kapillar-Drainageschlauch-System nach Fig. 4,
- Fig. 6: einen Querschnitt des Kapillar-Drainageschlauch-Systems entlang einer Schnittebene B-B nach Fig. 4,
- Fig. 7: einen Querschnitt des modifizierten Kapillar-Drainageschlauch-Systems entlang einer Schnittebene C-C nach Fig. 5.

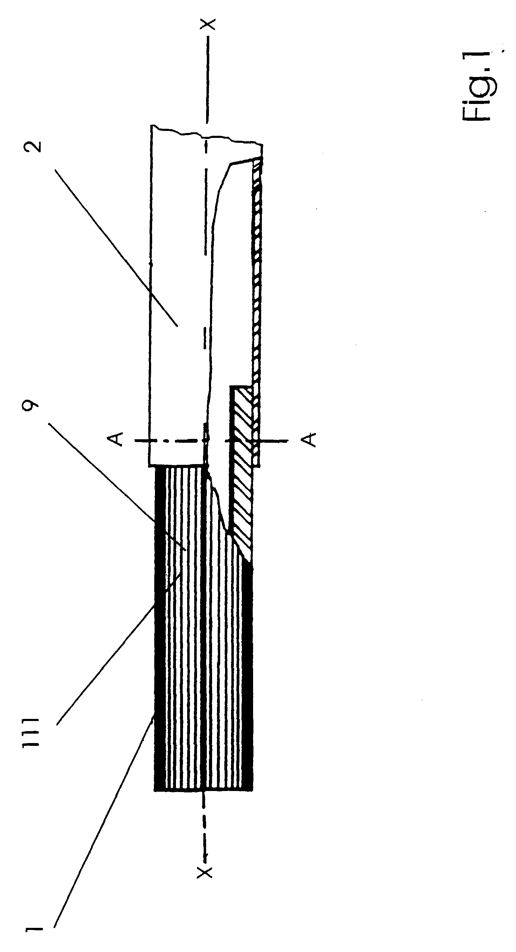

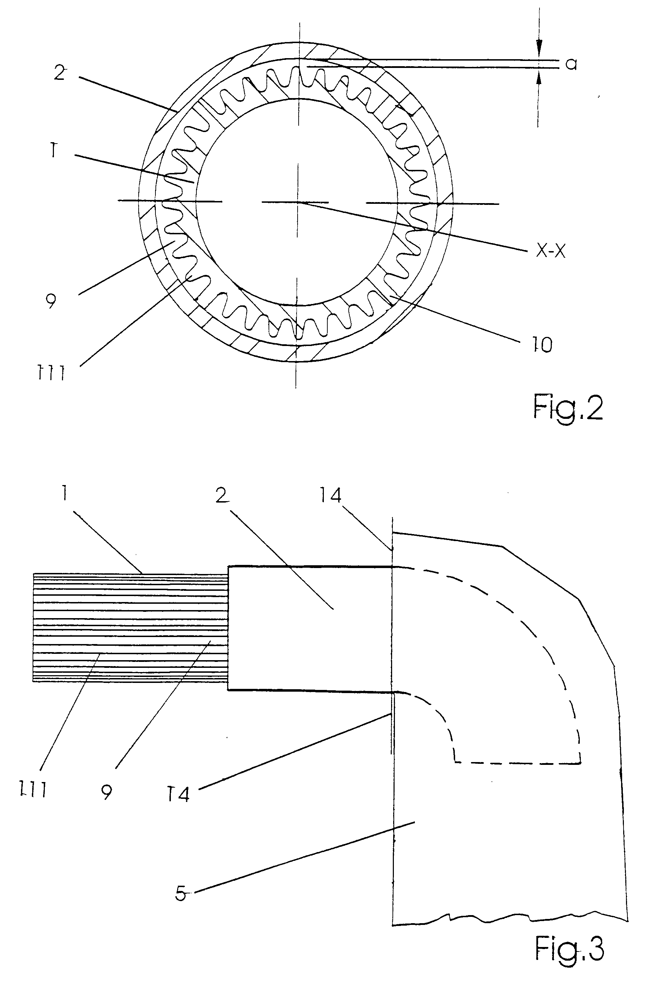

Bei dem Kapillar-Drainageschlauch-System für die Abführung von Körper- oder Wundsekreten wird, wie in Fig. 1 dargestellt, von einem Schlauch 2 ausgegangen. In das distale Ende dieses Schlauches 2 ist ein dieses Ende überragender Kapillarschlauch 1 eingeführt, wobei dem Kapillarschlauch 1 wenigstens auf seiner äußeren Oberfläche über den Umfang verteilt angeordnete, in Längserstreckungsrichtung zum Kapillarschlauch 1 verlaufende äußere Stege 111 gegeben sind, die voneinander derart beabstandet sind, daß sie in verlegten Zustand an ihrem äußeren Umfang vom Körpergewebe im wesentlichen umfaßt sind und somit ein Kapillarprofil 9 bilden, wodurch in Folge der wirkenden Kapillarkräfte die Sekrete aus dem umgebenden Gewebe des Kapillarschlauchs 1 abgesaugt werden.
Das Kapillarprofil 9 wird, wie in Fig. 2 gezeigt, durch die äußeren Stege 111 des Kapillarschlauchs 1 innerhalb des Schlauchs 2 fortgesetzt. Der Kapillarschlauch 1 trägt dabei zwischen den Stegen 111 verteilt äußere, in radialer Richtung eine größere Erstreckung von der Mittelachse X-X aufweisende, Profilstege 10. Über diese äußeren Profilstege 10 ist die Verbindung zu dem aufgeschobenen Schlauch 2 hergestellt, so daß zwischen dem Kapillarprofil 9 und der Innenwand des aufgeschobenen Schlauches 2 ein Abstand a verbleibt, der das Sekret in den Schlauch 2 einfließen läßt. Die Anzahl der Profilstege 10 ist so bemessen, daß die Beabstandung und Befestigung des Kapillarschlauchs 1 in Schlauch 2 auch beim Aufschrumpfen des Kapillar-Drainageschlauch-Systems erhalten bleibt.

Die Darstellung des Kapillar-Drainageschlauch-Systems in der Fig. 3 zeigt den mit dem Schlauch 2 verbundenen Kapillarschlauch 1, wobei dem proximalen Ende des Schlauches 2 ein Sekretbeutel 5 zugeordnet ist, wodurch das aus dem Schlauch 2 abfließende Sekret in dem Sekretbeutel 2 aufgefangen wird. Der Sekretbeutel 5 ist mit Klebestreifen 14 versehen, so daß dieser direkt an die Haut des Patienten fixiert werden kann.

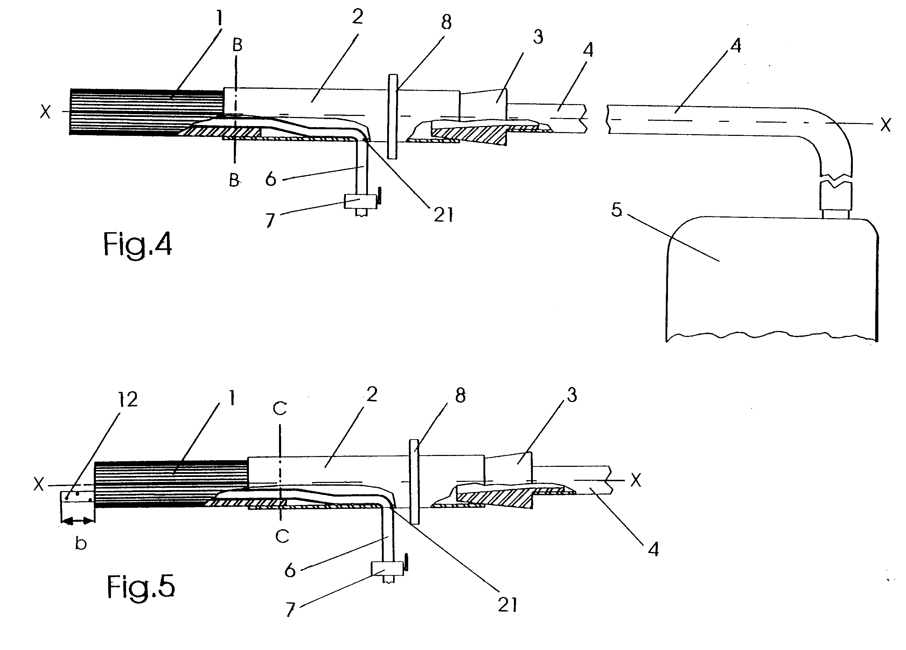

In Fig. 4 ist ein Kapillar-Drainageschlauch-System mit angeschlossenen Elementen dargestellt. In diesem Fall ist der mit dem Kapillarschlauch 1 verbundene Schlauch 2 mittels eines Konnektors 3 mit einem weiteren Verbindungsschlauch 4 verbunden, der an einen Sekretbeutel 5 angeschlossen ist. Darüber hinaus ist in dem Kapillarschlauch 1 und weiterführend in dem Schlauch 2 wenigstens ein Zuführungsschlauch 6 fest oder lose angeordnet. Dieser Zuführungsschlauch 6 ist durch einen seitlichen Durchbruch 21 dichtend aus dem Schlauch 2 nach außen geführt. Außerhalb des Schlauchs 2 ist dem Zuführungsschlauch 6 eine erste Absperrvorrichtung 7, bspw. ein Absperrventil, zugeordnet. Desweiteren besitzt der Schlauch 2 zwischen dem Durchbruch 21 und dem Konnektor 3 eine zweite Absperrvorrichtung 8, bspw. eine Schlauchklemme, so daß bei geöffneter erster Absperrvorrichtung 7 und geschlossener zweiter Absperrvorrichtung 8 ein Spülmittel oder anderes Behandlungsmittel durch den Zuführungsschlauch 6 in die Wunde gelangen kann bzw. bei geschlossener erster Absperrvorrichtung 7 und geöffneter zweiter Absperrvorrichtung 8 das Wundsekret, Spülmittel oder andere Behandlungsmittel durch den Kapillarschlauch 1 über den Schlauch 2, den Konnektor 3 und den Verbindungsschlauch 4 in den Sekretbeutel 5 abgeführt werden.

Die Fig. 5 zeigt eine Variante des Kapillar-Drainageschlauch-Systems, bei der der Zuführungsschlauch 6 in einer Länge b über den Kapillarschlauch 1 hinausragt. In diesem Bereich sind seitliche Ausnehmungen 12 angeordnet, so daß die durch den Zuführungsschlauch 6 in die Wunde gleiteten Flüssigkeiten gleichmäßiger in das umgebende Gewebe abgegeben werden.

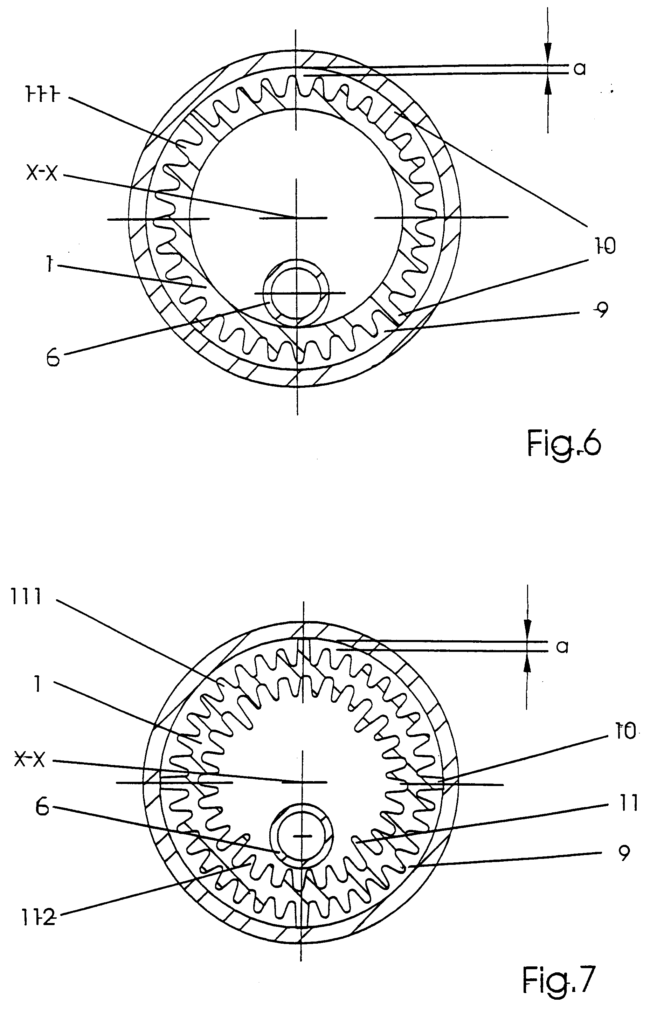

Die Fig. 6 zeigt den Querschnitt des Kapillar-Drainageschlauch-Systems entlang einer Schnittebene B-B. In diesem Fall trägt der Kapillarschlauch 1 in seinem Inneren den Zuführungsschlauch 6, der bspw. fest mit dem Kapillarschlauch 1 verbunden ist, und an seiner Außenwand äußere Stege 111, wobei zwischen den Stegen 111 äußere Profilstege 10 angeordnet sind. Über diese äußeren Profilstege 10 ist die Verbindung zu dem Schlauch 2 hergestellt, so daß zwischen dem Kapillarprofil 9 und der Innenwand des Schlauches 2 ein Abstand a entsteht, der das Sekret in den Schlauch 2 einfließen läßt. Die Anzahl der Profilstege 10 ist so bemessen, daß die Beabstandung und Befestigung des Kapillarschlauchs 1 in Schlauch 2 auch beim Aufschrumpfen des Kapillar-Drainageschlauch-Systems erhalten bleibt.

Die Fig. 7 zeigt den Querschnitt des modifizierten Kapillar-Drainageschlauch-Systems entlang der Schnittebene C-C. In diesem Fall trägt der Kapillarschlauch 1 in seinem Inneren den Zuführungsschlauch 6, der bspw. lose gelagert ist. Darüber hinaus weist der Kapillarschlauch 1, neben den äußeren Stegen 111 auf seiner äußeren Oberfläche, auf seiner inneren Oberfläche über den Umfang verteilt angeordnete, in Längserstreckungsrichtung zum Kapillarschlauch 1 verlaufende innere Stege 112 auf, wobei zwischen den äußeren Stegen 111 bzw. den inneren Stegen 112 verteilt die äußeren Profilstege 10 bzw. innere, in radialer Richtung eine größere Erstreckung zur Mittelachse X-X des Kapillarschlauchs 1 aufweisend Profilstege 11 angeordnet sind. Über die äußeren Profilstege 10 ist die Verbindung zu dem Schlauch 2 hergestellt. Diese Verbindung hat den Vorteil, daß zwischen dem Kapillarprofil 9 und der Innenwand des Schlauches 2 ein Abstand a entsteht, der das Sekret ohne Behinderung in den Schlauch 2 einfließen läßt. Die inneren Profilstege 11 sind so bemessen, daß der Kapillarschlauch 1 beim Zusammendrücken nicht kollabiert, so daß Sekret in seinem Abfluß nicht gehemmt wird. Die Anzahl der Profilstege 10 und 11 ist dabei so festgelegt, daß die Beabstandung und Befestigung des Kapillarschlauchs 1 beim Aufschrumpfen gewährleistet ist.

Der Vorteil des erfindungsgemäßen Kapillar-Drainageschlauch-Systems besteht insbesondere darin, daß durch die bewirkten Kapillarkräfte das Wundsekret aufgrund des Kapillarprofils 9 allseitig gut aus der Wunde abfließen kann. Darüber hinaus ist von Vorteil, daß vermittels des Zuführungsschlauchs 6 der Wunde Mittel zum Spülen, Medikamente etc. zugeführt werden können und daß das Kapillar-Drainageschlauch-System problemlos ohne eine zusätzliche Wundbehandlung aus der Wunde entfernbar ist.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichenliste

- 1: - Kapillarschlauch
- 2: - Schlauch
- 21: - Durchbruch
- 3: - Konnektor
- 4: - Verbindungsschlauch
- 5: - Sekretbeutel
- 6: - Zuführungsschlauch
- 7: - erste Absperrvorrichtung
- 8: - zweite Absperrvorrichtung
- 9: - Kapillarprofil
- 10: - äußerer Profilsteg
- 11: - innerer Profilsteg
- 111: - äußere Stege
- 112: - innere Stege
- 12: - seitliche Ausnehmungen
- 14: - Klebestreifen
- a: - Abstand
- b: - Länge
- A-A: - Schnittebene
- B-B: - Schnittebene
- C-C: - Schnittebene
- X-X: - Mittelachse

## Patentansprüche

1. Kapillar-Drainageschlauch-System für die Abführung von Körper- oder Wundsekreten, umfassend wenigstens einen Schlauch (2), dadurch gekennzeichnet, daß in das distale Ende des Schlauches (2) ein dieses Ende überragender Kapillarschlauch (1) eingeführt ist, wobei dem Kapillarschlauch (1) wenigstens auf seiner äußeren Oberfläche über den Umfang verteilt angeordnete, in Längserstreckungsrichtung zum Kapillarschlauch (1) verlaufende äußere Stege (111) gegeben sind, die voneinander derart beabstandet sind, daß sie in verlegten Zustand an ihrem äußeren Umfang vom Körpergewebe im wesentlichen umfaßt sind und somit ein Kapillarprofil (9) bilden.

2. Kapillar-Drainageschlauch-System nach Anspruch 1, dadurch gekennzeichnet, daß ein Teil der äußeren Stege (111) in radialer Richtung eine größere Erstreckung von der Mittelachse X-X des Kapillarschlauchs (1) aufweisen, als die übrigen, so daß äußere Profilstege (10) gebildet sind, die den Kapillarschlauch (1) mit einem Abstand (a) in dem Schlauch (2) haltern.

3. Kapillar-Drainageschlauch-System nach den Ansprüchen 1 und 2, dadurch gekennzeichnet, daß dem Kapillarschlauch (1) auf seiner inneren Oberfläche über den Umfang verteilt angeordnete, in Längserstreckungsrichtung zum Kapillarschlauch (1) verlaufende innere Stege (112) gegeben sind.

4. Kapillar-Drainageschlauch-System nach den Ansprüchen 1 und 3, dadurch gekennzeichnet, daß ein Teil der inneren Stege (112) in radialer Richtung eine größere Erstreckung zur Mittelachse X-X des Kapillarschlauchs (1) aufweisen, als die übrigen, so daß innere Profilstege (11) gebildet sind.

5. Kapillar-Drainageschlauch-System nach den Ansprüchen 1 bis 4, dadurch gekennzeichnet, daß der Kapillarschlauch (1) im Inneren mit zumindest einen Zuführungsschlauch (6) für liquide Medien versehen ist.

6. Kapillar-Drainageschlauch-System nach den Ansprüchen 1 und 5, dadurch gekennzeichnet, daß der Zuführungsschlauch (6) das distale Ende des Kapillarschlauchs (1) um eine Länge (b) überragt.

7. Kapillar-Drainageschlauch-System nach den Ansprüchen 1 und 6, dadurch gekennzeichnet, daß der Zuführungsschlauch (6) in Bereich des überragenden Endes mit seitlichen Ausnehmungen (12) versehen ist.

8. Kapillar-Drainageschlauch-System nach Anspruch 1, dadurch gekennzeichnet, daß das proximale Ende des Schlauchs (2) mit einem Sekretbeutel (5) verbunden ist.

9. Kapillar-Drainageschlauch-System nach den Ansprüchen 1 und 8, dadurch gekennzeichnet, daß der Schlauch (2) mittels eines Konnektors (3) und eines weiteren Verbindungsschlauchs (4) mit dem Sekretbeutel (5) in Verbindung gebracht ist.

10. Kapillar-Drainageschlauch-System nach den Ansprüchen 1, 8 und 9, dadurch gekennzeichnet, daß das der Sekretbeutel (5) mit Klebestreifen (14) zwecks Befestigung an der Haut des Patienten versehen ist.

11. Kapillar-Drainageschlauch-System nach den Ansprüchen 1 und 5, dadurch gekennzeichnet, daß der Zuführungsschlauch (6) dichtend durch einen seitlichen Durchbruch (21) aus dem Schlauch (2) nach außen geführt ist.

12. Kapillar-Drainageschlauch-System nach den Ansprüchen 1 und 11, dadurch gekennzeichnet, daß der Zuführungsschlauch (6) in einem Abschnitt außerhalb des Schlauches (2) mit einer ersten Absperrvorrichtung (7) und der Schlauch (2) zwischen dem Durchbruch (21) und dem Konnektor (3) mit einer zweiten Absperrvorrichtung (8) versehen ist.
